**Europäisches Patentamt**

**European Patent Office**

**Office européen des brevets**

(11) Numéro de publication: **0 060 771**
**B1**

(12) **FASCICULE DE BREVET EUROPEEN**

(45) Date de publication du fascicule du brevet:
**03.07.85**

(21) Numéro de dépôt: **82400416.2**

(22) Date de dépôt: **09.03.82**

(51) Int. Cl.⁴: **H 05 H 7/12,** G 21 K 1/08,
A 61 N 5/10

(54) Dispositif de radiographie utilisant l'accélérateur de particules chargées d'un appareil de radiothérapie, et appareil de radiothérapie équipé d'un tel dispositif.

(30) Priorité: **13.03.81 FR 8105138**

(43) Date de publication de la demande:
**22.09.82 Bulletin 82/38**

(45) Mention de la délivrance du brevet:
**03.07.85 Bulletin 85/27**

(84) Etats contractants désignés:
**DE GB**

(56) Documents cités:
**EP - A - 0 041 753**
**FR - A - 2 357 989**
**FR - A - 2 466 084**
**US - A - 3 582 650**
**US - A - 3 867 635**

(73) Titulaire: **C.G.R. MeV, Route de Guyancourt,
F-78530 Buc (FR)**

(72) Inventeur: **Azam, Guy, THOMSON-CSF SCPI 173, bld
Haussmann, F-75360 Paris Cedex 08 (FR)**

(74) Mandataire: **Barbin le Bourhis, Joel et al,
THOMSON-CSF SCPI 173, boulevard Haussmann,
F-75379 Paris Cedex 08 (FR)**

ACTORUM AG

## Description

La présente invention a pour objet un dispositif de radiographie utilisant l'accélérateur de particules chargées, des électrons par exemple, d'un appareil de radiothérapie, ce dispositif étant destiné à permettre la vérification du positionnement de la zone à irradier par rapport à l'axe du faisceau d'irradiation.

Un tel dispositif est plus particulièrement destiné au traitement de radiothérapie, le positionnement de la zone à traiter devant, dans tous les cas, être réalisé de façon très précise.

La vérification du positionnement de la zone à traiter peut s'effectuer, de façon connue, à partir de repères tracés sur cette zone, mais cette méthode reste imprécise, si l'on tient compte de l'évolution possible de la morphologie du patient au cours du traitement de radiothérapie qui peut, en fait, durer plusieurs semaines.

Il est également connu d'utiliser, pour la vérification du bon positionnement du malade à traiter, un générateur de rayons X qui peut être fixé sur la tête d'irradiation, comme cela a été décrit dans l'article de R. WIDEROE concernant un Asklépitron (BROWN, BOVERI & CIE), soit sur un support placé à l'intérieur de la tête d'irradiation comme décrit dans la demande de brevet français FR-A-2 466 084 de la Demanderesse.

Dans d'autres appareils d'irradiaton, la vérification du positionnement de la zone à traiter est obtenue au moyen d'un faisceau «flash» de rayons X à haute énergie. Mais la cliché obtenu de cette façon, à partir du faisceau nominal délivré par l'accélérateur, n'est pas de qualité satisfaisante et l'exploitation de ce cliché présente des difficultés.

Le dispositif suivant l'invention ne présente pas ces inconvénients et permet d'obtenir des images radiographiques de vérification de positionnement de la zone à traiter d'excellente qualité.

Suivant l'invention, un dispositif de radiographie utilisant l'accélérateur de particules chargées d'un appareil de radiothérapie destiné à fournir un faisceau d'irradiation d'axe YY, cet appareil comportant un accélérateur de particules chargées muni d'une source de particules chargées destinée à fournir un faisceau $f_1$ de particules chargées faiblement accélérées, une structure accélératrice permettant d'obtenir un faisceau $f_3$ de particules chargées fortement accélérées, est caractérisé en ce qu'il comporte un dispositif de déviation magnétique de particules chargées, et au moins une première cible destinée à être placée au moins temporairement de manière à recevoir le faisceau de particules faiblement accéléré et à émettre suivant l'axe YY un faisceau $f_2$ de rayons X à basse énergie.

L'invention concerne également un appareil de radiothérapie destiné à fournir suivant un axe YY un faisceau d'irradiation, du type décrit par exemple dans le document FR-A-2 357 989 et comportant un accélérateur de particules muni d'une source de particules chargées destinée à fournir un fasceau de particules chargées faiblement accélérées, une structure accélératrice permettant d'obtenir un faisceau de particules chargées fortement accélérées, et un dispositif de déviation magnétique de particules chargées, caractérisé en ce qu'il comprend en outre au moins une première cible destinée à être placée au moins temporairement de manière à recevoir le faisceau de particules faiblement accélérées et à émettre suivant l'axe YY, un faisceau de rayons X à basse énergie.

L'invention sera mieux comprise et d'autres caractéristiques apparaîtront à l'aide de la description ci-après et des dessins qui l'accompagnent sur lesquels:

Les figures 1 à 3 représentent schématiquement trois exemples de réalisation d'un dispositif suivant l'invention.

La figure 4 montre un exemple de réalisation d'une cible.

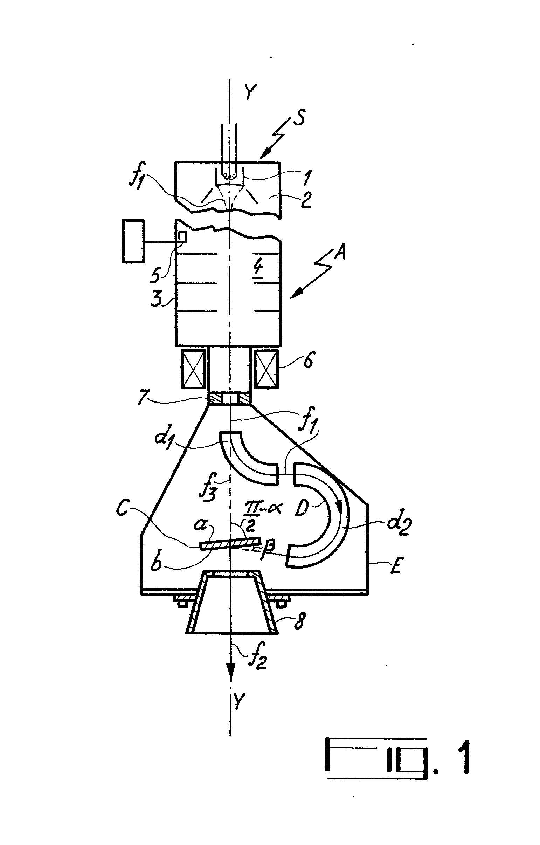

Dans le premier exemple de réalisation montré en figure 1, le dispositif suivant l'invention comprend un accélérateur A de particules (un accélérateur linéaire par exemple) muni d'une source S de particules (canon à électrons par exemple, comprenant une cathode 1, des électrodes 2 ... de types connus), une structure 3 accélératrice constituée d'une succession de cavités résonnantes 4 à microondes, des moyens de couplage 5 permettant l'injection d'une énergie microonde dans la structure 3 accélératrice, des bobines 6 de focalisation du faisceau d'électrons issu de la cathode 1, un collimateur primaire 7.

A la sortie de la structure 3 accélératrice est disposé un dispositif D de déviation magnétique destiné à dévier le faisceau $f_1$ d'électrons issus de la cathode 1 et non accélérés dans la structure 3 accélératrice.

Ce dispositif D de déviation magnétique comprend, dans l'exemple de réalisation montré en figure 1, un déviateur $d_1$ destiné à dévier le faisceau $f_1$ d'un angle $\pi/2$ (sens direct) suivi d'un déviateur $d_2$ destiné à dévier le faisceau $f_1$ émergeant du déviateur $d_1$, d'un angle $\pi + \beta$ en sens opposé.

Les déviateurs $d_1$, $d_2$ sont formés d'électro-aimants munis chacun d'une paire de pièces polaires disposées en vis-à-vis et délimitant des entrefers dans lesquels peut passer le faisceau $f_1$ d'électrons.

Le dispositif suivant l'invention comporte enfin une cible C en tungstène par exemple, disposée sur l'axe YY, le plan de la surface de cette cible C étant incliné d'un angle $\alpha$ par rapport à la normale à cet axe YY, de telle sorte que le faisceau $f_1$ non accéléré dans la structure 3, émergeant du déviateur $d_2$ puisse venir frapper cette cible C sensiblement en un point O intersection de la cible C et de l'axe YY.

L'ensemble formé par le déviateur D et la cible C est placé dans une enceinte E étanche au vide et rendue solidaire de l'accélérateur A.

En fonctionnement, le canon à électrons peut être soumis à une tension de quelques kvolts, par exemple 80 kvolts environ qui confère aux électrons une accélération d'origine. Dans ces conditions, il émet un faisceau $f_1$ d'électrons faiblement accéléré par cette accélération d'origine, destiné à traverser la structure 3 accélératrice.

Dans un premier temps, l'énergie microonde n'étant pas injectée dans la structure 3, le faisceau $f_1$ faiblement accéléré traverse la structure 3 sans être accéléré dans la structure accélératrice. A la sortie de la structure 3, le faisceau $f_1$ faiblement accéléré pénètre dans l'entrefer des pièces polaires des électro-aimants formant le dispositif D de déviation qui est mis en fonctionnement. Le faisceau $f_1$ subit une première déviation d'un angle $\gamma_1 = + \frac{\pi}{2}$, dans le premier déviateur $d_1$, puis une seconde déviation d'un angle $\gamma_2 = - (\pi + \beta)$ dans le second déviateur $d_2$, $\beta$ étant supérieur ou égal à $\alpha$. Le faisceau $f_1$, émergeant du déviateur $d_2$ vient frapper la face b de la cible C suivant un angle d'incidence $\beta$. Cette cible C émet alors, par cette face b, un faisceau $f_2$ de rayons X à basse énergie permettant d'obtenir un cliché radiographique de la zone à irradier et donc de vérifier le positionnement convenable de la zone à irradier par rapport à l'axe YY correspondant à la trajectoire moyenne du faisceau d'irradiation.

Lorsque cette vérification est effectuée, la tension d'alimentation des déviateurs $d_1$ et $d_2$ est supprimée et l'accélérateur A mis en fonctionnement. Cet accélérateur A délivre un faisceau $f_3$ de particules, dites fortement accélérées par opposition au faisceau $f_1$ de particules faiblement accélérées, qui vient frapper la face a de la cible C.

Sous l'impact du faisceau $f_3$, la cible C émet, par sa face b, un faisceau $f_4$ de rayons X à haute énergie, destinée à l'irradiation de la zone à traiter, la trajectoire moyenne du faisceau $f_4$ coïncidant avec l'axe YY.

Ce faisceau $f_4$ peut être collimaté, de façon connue, au moyen d'un système de collimation 8 à éléments mobiles, permettant la délimitation convenable du faisceau $f_4$ d'irradiation. Le cliché radiographique obtenu au moyen du faisceau $f_2$ de rayons X à basse énergie permet de vérifier, en plus du centrage de la zone à irradier, la surface de cette zone qui sera irradiée, telle que délimitée par le système de collimation.

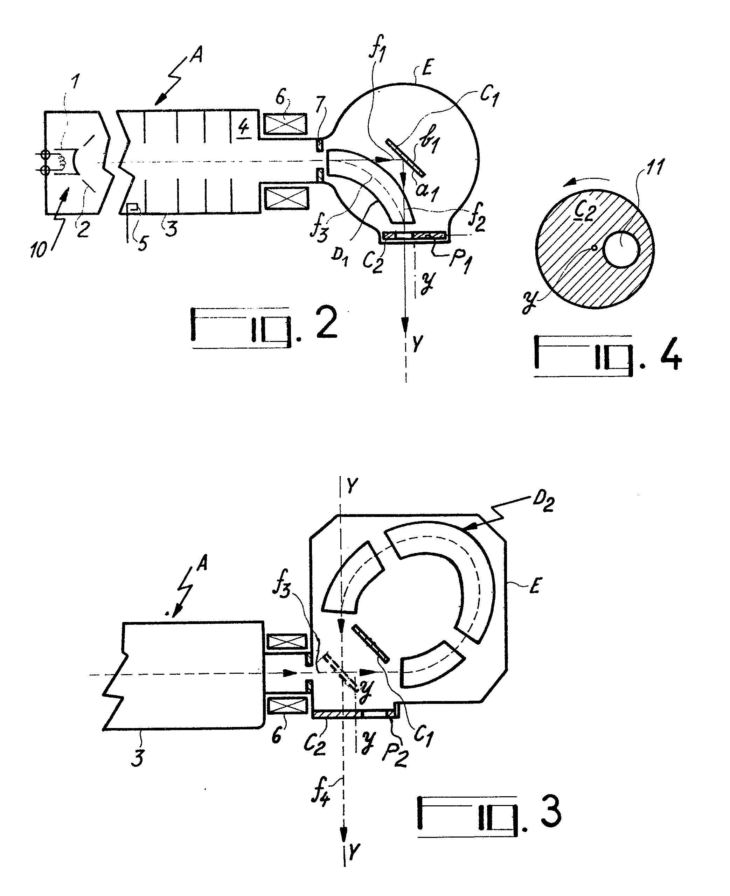

La figure 2 montre un deuxième exemple de réalisation d'un dispositif suivant l'invention dans lequel l'axe YY fait un angle de 90° avec l'axe du faisceau $f_1$, de particules faiblement accélérées arrivant sur la cible C; cette dernière étant représentée sur la figure 2 par une cible $C_1$ escamotable.

Ce dispositif comprend un accélérateur A de particules muni d'une source de particules chargées (canon 10 à électrons par exemple), une structure 3 accélératrice permettant d'accélérer le faisceau $f_1$ de particules émis par le canon 10, et de délivrer un faisceau $f_3$ de particules fortement accélérées.

Un dispositif $D_1$ de déviation est disposé à la sortie de l'accélérateur dans une enceinte E à vide et permet de dévier de $\pi/2$ le faisceau $f_3$ de particules fortement accélérées.

Une cible $C_1$ escamotable, en tungstène par exemple, est placée dans l'enceinte E à vide en vis-à-vis de la sortie de l'accélérateur A de telle sorte que la face $a_1$ de la cible $C_1$ puisse émettre, sous l'impact du faisceau $f_1$ de particules chargées, faiblement accélérées, un faisceau $f_2$ de rayons X à basse énergie, ce faisceau $f_2$ ayant pour axe, l'axe YY du faisceau de traitement. Le faisceau $f_2$ de rayons X, comme dans l'exemple précédent, permet d'obtenir un cliché radiographique de la zone à traiter et donc de vérifier son positionnement avant irradiation et la surface qui sera effectivement traitée.

Lorsque cette étape de vérification est achevée, le dispositif $D_1$ de déviation est mis sous tension et l'accélérateur est mis en fonctionnement, délivrant un faisceau $f_3$ de particules chargées fortement accélérées qui est dévié dans le dispositif $D_1$ de façon que, à la sortie de ce dernier, sa trajectoire moyenne viennent coïncider avec l'axe YY d'irradiation.

Le faisceau de traitement peut être le faisceau $f_3$ lui-même, ou un faisceau $f_4$ de rayons X à haute énergie, obtenu en interposant sur le trajet du faisceau $f_3$ une seconde cible $C_2$ mobile. Sous l'impact du faisceau $f_3$, la cible $C_2$ peut émettre, de façon connue, le faisceau $f_4$ de rayons X à haute énergie.

Dans l'exemple de réalisation montré en figure 2, la cible $C_2$ (voir figure 4) en tungstène par exemple, est de forme circulaire, d'axe yy. Elle comporte un orifice 11 excentré. Par rotation, la cible $C_2$ peut occuper une première position $P_1$ dans laquelle l'axe de l'orifice 11 coïncide avec l'axe YY. Dans ce cas, le faisceau de traitement est le faisceau $f_3$ de particules fortement accélérées.

Dans une seconde position $P_2$ de la cible $C_2$, l'orifice 11 est placé hors de l'axe YY et cette cible $C_2$ peut accomplir sa fonction de cible et être bombardée par le faisceau $f_3$. Dans ce cas, le faisceau de traitement est le faisceau $f_4$ de rayons X à haute énergie.

Les exemples de réalisation d'un dispositif suivant l'invention, montrés en figures 1 et 2, ne sont pas limitatifs.

En particulier, le dispositif de déviation peut être d'un autre type que ceux qui ont été précédemment décrits.

La figure 3 représente un autre exemple de réalisation d'un dispositif suivant l'invention. Dans ce cas l'axe YY fait également un angle de 90° avec l'axe du faisceau $f_1$ de particules faiblement accélérées. Le dispositif de déviation $D_2$ du type « $\frac{3\pi}{2}$ » destiné à dévier d'un angle $3\pi/2$, le faisceau $f_3$ de particules fortement accélérées issu de l'accélérateur A. Ce faisceau $f_3$, comme dans les exemples précédemment décrits, peut être utilisé directement comme faisceau de traitement ou peut être utilisé pour bombarder une cible $C_2$ qui peut alors émettre un faisceau $f_4$ de traitement à rayon X de haute énergie. Une cible $C_1$, placée dans l'enceinte E à vide, et associée à des moyens de déplacement (non représentés sur la figure), peut être disposée temporairement sur le trajet du faisceau $f_1$ de particules faiblement accélérées sortant de l'accélérateur A. Cette cible escamotable $C_1$ placée dans cette configuration est représentée en pointillé sur la figure 4. Parallèlement la cible $C_2$ est orientée de telle sorte que l'orifice 11 se trouve placé dans l'axe YY. Dans ces conditions on obtient un faisceau $f_2$ de rayon X à basse énergie permettant de réaliser un cliché radiographique, d'excellente qualité, de la zone à traiter. Après vérification du positionnement, la cible mobile $C_1$ est escamotée et le traitement peut être effectué soit avec un faisceau $f_3$ de particules fortement accélérées si l'orifice 11 de la cible $C_2$ est placé dans l'axe YY soit au moyen d'un faisceau $f_4$ de rayons X à haute énergie si cet orifice est placé en dehors de cet axe YY.

L'invention s'applique à tout appareil de radiothérapie utilisant un accélérateur de particules chargées équipé de ce dispositif de radiographie conforme à l'invention.

## Revendications

1. Dispositif de radiographie utilisant l'accélérateur de particules chargées d'un appareil de radiothérapie destiné à fournir suivant un axe YY un faisceau d'irradiation, cet appareil comportant un accélérateur (A) de particules muni d'une source (S) de particules chargées destinée à fournir un faisceau ($f_1$) de particules chargées faiblement accélérées, une structure accélératrice (A) permettant d'obtenir un faisceau ($f_3$) de particules chargées fortement accélérées, caractérisé en ce qu'il comprend un dispositif de déviation magnétique (D) de particules chargées et au moins une première cible (C), destinée à être placée au moins temporairement de manière à recevoir le faisceau ($f_1$) de particules faiblement accélérées et à émettre suivant l'axe YY, un faisceau ($f_2$) de rayons X à basse énergie.

2. Dispositif suivant la revendication 1, caractérisé en ce que cette première cible est une cible fixe (C) placée sur l'axe YY, le plan de la surface de ladite cible étant incliné d'un angle $\alpha$ par rapport à la normale à cet axe YY, la cible (C) étant destinée dans un premier temps à être bombardée, par le faisceau ($f_1$) de particules chargées faiblement accélérées, de façon à émettre le faisceau ($f_2$) de rayons à basse énergie et, dans un second temps par le faisceau ($f_3$) de particules chargées fortement accélérées, de façon à émettre un faisceau ($f_4$) de rayons X à haute énergie.

3. Dispositif suivant la revendication 2, caractérisé en ce que le dispositif de déviation magnétique (D) comprend au moins deux déviateurs ($d_1$, $d_2$) prévus pour dévier le faisceau ($f_1$) de particules chargées faiblement accélérées issu de l'accélérateur (A) successivement d'un angle $\gamma_1$, puis d'un angle $\gamma_2$, de sens opposés.

4. Dispositif suivant la revendication 3, caractérisé en ce que l'angle d'incidence ($\beta$) du faisceau ($f_1$) dévié, arrivant sur la cible (C), est supérieur ou égal à $\alpha$.

5. Appareil de radiothérapie destiné à fournir suivant un axe YY un faisceau d'irradiation, comportant un accélérateur (A) de particules muni d'une source (S) de particules chargées destinée à fournir un faisceau ($f_1$) de particules chargées, faiblement accélérées, une structure accélératrice (3) permettant d'obtenir un faisceau ($f_3$) de particules chargées fortement accélérées, et un dispositif de déviation magnétique (D) de particules chargées caractérisé en ce qu'il comprend en outre au moins une première cible (C), destinée à être placée au moins temporairement de manière à recevoir le faisceau ($f_1$) de particules faiblement accélérées et à émettre suivant l'axe YY un faisceau ($f_2$) de rayons X à basse énergie.

6. Appareil suivant la revendication 5, caractérisé en ce que cette première cible est une cible fixe (C) placée sur l'axe YY, le plan de la surface de ladite cible étant incliné d'un angle $\alpha$ par rapport à la normale à cet axe YY, la cible (C) étant destinée dans un premier temps à être bombardée, par le faisceau ($f_1$) de particules chargées faiblement accélérées, de façon à émettre le faisceau ($f_2$) de rayons X à basse énergie et, dans un second temps par le faisceau ($f_3$) de particules chargées fortement accélérées, de façon à émettre le faisceau ($f_4$) de rayons X à haute énergie.

7. Appareil suivant la revendication 6, caractérisé en ce que le dispositif de déviation magnétique (D) comprend au moins deux déviateurs ($d_1$, $d_2$) prévus pour dévier le faisceau ($f_1$) de particules chargées faiblement accélérées issu de l'accélérateur (A) successivement d'un angle $\gamma_1$, puis d'un angle $\gamma_2$, de sens opposés.

8. Appareil suivant la revendication 7, caractérisé en ce que l'angle d'incidence ($\beta$) du faisceau ($f_1$) dévié, arrivant sur la cible (C), est supérieur ou égal à $\alpha$.

9. Appareil selon la revendication 5, caractérisé en ce que l'axe YY fait un angle de 90° avec l'axe du faisceau ($f_1$) de particules chargées non accélérées arrivant sur la cible (C).

10. Appareil selon la revendication 9, caractérisé en ce que le dispositif (D) de déviation est du type $\pi$ 2, l'axe du faisceau ($f_3$) de particules chargées à sa sortie du dispositif (D) de déviation étant alors confondu avec l'axe YY.

11. Appareil selon la revendication 9, caractérisé en ce que le dispositif (D) de déviation est du type $3\pi/2$, l'axe du faisceau ($f_3$) de particules chargées à sa sortie du dispositif (D) de déviation étant alors confondu avec l'axe YY.

12. Appareil selon l'une des revendications 9 ou 10, caractérisé en ce que la première cible est une cible escamotable ($C_1$) capable d'être positionnée sur le parcours du faisceau ($f_1$) pour émettre le faisceau ($f_2$) de rayons X à basse énergie, orienté selon l'axe YY.

13. Appareil selon l'une des revendications 10 à 12, caractérisé en ce qu'il comporte en outre une seconde cible ($C_2$) placée sur l'axe YY et capable d'occuper deux positions ($P_1$) et ($P_2$).

14. Appareil selon la revendication 13, caractérisé en ce que cette cible ($C_2$) est de forme circulaire et comporte un orifice (11) excentré tel qu'en position ($P_2$) elle accomplisse sa fonction de cible et en position ($P_1$), l'axe de l'orifice (11), coïncidant avec l'axe YY elle laisse passer librement tout faisceau émis selon cet axe.

15. Appareil selon l'une des revendications 13 ou 14, caractérisé en ce que la cible ($C_2$) étant en position ($P_1$), la cible ($C_1$) est positionnée sur le

parcours du faisceau ($f_1$) et émet un faisceau ($f_2$) de rayons X à basse énergie selon l'axe YY.

16. Appareil selon l'une des revendications 13 ou 14, caractérisé en ce que la cible ($C_1$) étant escamotée, la cible ($C_2$) est en position ($P_1$) et le faisceau de particules chargées fortement accélérées ($f_3$) est émis.

17. Appareil selon l'une des revendications 13 ou 14, caractérisé en ce que la cible ($C_1$) étant escamotée, la cible ($C_2$) est en position ($P_2$), le faisceau ($f_3$) est émis et bombardant la cible ($C_2$) entraîne l'émission d'un faisceau ($f_4$) de rayons X à haute énergie.

**Patentansprüche**

1. Den Beschleuniger ladungstragender Massenteilchen eines längs einer YY-Achse ein Strahlenbündel abgebenden Röntgenbestrahlungsgerätes verwendende Röntgenvorrichtung, wobei dieses Gerät einen Massenteilchenbeschleuniger (A) mit einer Quelle (S) geladener Teilchen aufweist, die ein Bündel ($f_1$) schwach beschleunigter geladener Teilchen abgibt, und wobei vermittels einer Beschleunigerstruktur (A) ein Bündel ($f_3$) stark beschleunigter geladener Teilchen erzielbar ist, dadurch gekennzeichnet, dass sie eine Vorrichtung (D) zur magnetischen Ablenkung geladener Teilchen, sowie wenigstens ein erstes Ziel (C) aufweist, das wenigstens zeitweise derart positionierbar ist, dass es das Bündel ($f_1$) schwach beschleunigter Teilchen empfängt und längs der YY-Achse ein Röntgenstrahlenbündel ($f_2$) mit geringer Energie abstrahlt.

2. Vorrichtung nach Anspruch 1, dadurch gekennzeichnet, dass dieses erste Ziel ein auf der YY-Achse angeordnetes ortsfestes Ziel ist, wobei die Oberflächenebene dieses Ziels in bezug auf die zur YY-Achse senkrechte Gerade unter einem Winkel $\alpha$ geneigt ist und das Ziel (C) zunächst von dem Bündel ($f_1$) schwach beschleunigter geladener Teilchen derart bombardierbar ist, dass es das Strahlenbündel ($f_2$) mit niedriger Energie abstrahlt, während das Ziel sodann von dem stark beschleunigten Teilchenbündel ($f_3$) derart bombardierbar ist, dass ein Röntgenstrahlenbündel ($f_4$) mit hoher Energie abstrahlt.

3. Vorrichtung nach Anspruch 2, dadurch gekennzeichnet, dass die magnetische Ablenkungsvorrichtung (D) wenigstens zwei Ablenkeinheiten ($d_1$, $d_2$) aufweist, durch die das aus dem Beschleuniger (A) austretende schwach beschleunigte Teilchenbündel ($f_1$) zunächst um einen Winkel $\gamma_1$ und dann um einen demselben entgegengesetzt orientierten Winkel $\gamma_2$ umlenkbar ist.

4. Vorrichtung nach Anspruch 3, dadurch gekennzeichnet, dass der Einfallswinkel ($\beta$) des abgelenkten Bündels ($f_1$) beim Auftreffen auf das Ziel (D) gleich oder grösser als $\alpha$ ist.

5. Röntgenbestrahlungsgerät zur Erzeugung eines Bestrahlungsbündels längs einer YY-Achse, mit einem Massenteilchenbeschleuniger (A), der Quelle (S) geladener Teilchen zum Abstrahlen eines Bündels ($f_1$) schwach beschleunigter geladener Teilchen besitzt, wobei durch eine Beschleunigerstruktur (3) ein Bündel ($f_3$) stark beschleuniger geladener Teilchen erzeugbar ist, während das Gerät ferner eine Vorrichtung (D) zur magnetischen Ablenkung geladener Teilchen aufweist, dadurch gekennzeichnet, dass es ebenfalls wenigstens ein erstes, wenigstens zeitweilig derart positionierbares Ziel (C) aufweist, dass es das schwach beschleunigte Teilchenbündel ($f_1$) empfängt und längs der YY-Achse ein Röntgenstrahlenbündel ($f_2$) mit niedriger Energie abstrahlt.

6. Gerät nach Anspruch 5, dadurch gekennzeichnet, dass dieses erste Ziel ein auf der YY-Achse angeordnetes ortsfestes Ziel (C) ist und die Oberflächenebene dieses Ziels gegen die zu dieser YY-Achse senkrechten Gerade unter einem Winkel $\alpha$ geneigt ist, wobei das Ziel (C) zunächst durch das Bündel ($f_1$) schwach beschleunigter geladener Teilchen derart bombardierbar ist, dass es das Strahlenbündel ($f_2$) mit niedriger Energie abstrahlt, und das Ziel sodann durch das Bündel ($f_3$) stark beschleunigter geladener Teilchen derart bombardierbar ist, dass es das Röntgenstrahlenbündel ($f_4$) mit hoher Energie abstrahlt.

7. Gerät nach Anspruch 6, dadurch gekennzeichnet, dass die magnetische Ablenkungsvorrichtung (D) wenigstens zwei Ablenkeinheiten ($d_1$, $d_2$) besitzt, durch die das aus dem Beschleuniger (A) austretende Bündel ($f_1$) schwach beschleunigter geladener Teilchen erst um einen Winkel $\gamma_1$ und sodann um einen demselben entgegengesetzt orientierten Winkel $\gamma_2$ umlenkbar ist.

8. Gerät nach Anspruch 7, dadurch gekennzeichnet, dass der Einfallswinkel ($\beta$) des abgelenkten, auf dem Ziel (C) auftreffenden Bündels ($f_1$) gleich oder grösser ist als $\alpha$.

9. Gerät nach Anspruch 5, dadurch gekennzeichnet, dass die YY-Achse mit der Achse des auf dem Ziel (C) auftreffenden Bündels ($f_1$) nicht beschleunigter geladener Teilchen einen Winkel von 90° bildet.

10. Gerät nach Anspruch 9, dadurch gekennzeichnet, dass die Ablenkungsvorrichtung (D) vom Typ $\pi 2$, ist, wobei die Achse des Bündels ($f_3$) geladener Teilchen bei dessen Austritt aus der Ablenkungsvorrichtung (D) mit der YY-Achse zusammenfällt.

11. Gerät nach Anspruch 9, dadurch gekennzeichnet, dass die Ablenkungsvorrichtung vom Typ $3\pi/2$ ist, wobei die Achse des Bündels ($f_3$) geladener Teilchen beim Austritt desselben aus der Ablenkungsvorrichtung (D) mit der YY-Achse zusammenfällt.

12. Gerät nach einem der Ansprüche 9 oder 10, dadurch gekennzeichnet, dass das erste Ziel ein einziehbares Ziel ($C_1$) und auf dem Strahlenweg des Bündels ($f_1$) positionierbar ist, um das in Richtung der YY-Achse orientierte Röntgenstrahlenbündel ($f_2$) mit niedriger Energie abzustrahlen.

13. Gerät nach einem der Ansprüche 10 bis 12, dadurch gekennzeichnet, dass es ferner ein auf der YY-Achse angeordnetes zweites Ziel ($C_2$) aufweist, welches in zwei Stellungen ($P_1$) und ($P_2$) bringbar ist.

14. Gerät nach Anspruch 13, dadurch gekennzeichnet, dass dieses Ziel ($C_2$) kreisförmig ist und eine exzentrische Öffnung (11) aufweist, derart, dass es in der Stellung ($P_2$) als Ziel wirksam ist und in der Stellung ($P_1$), in der die Achse der Öffnung (11) mit der YY-Achse zusammenfällt, jegliches in Richtung dieser Achse abgestrahlte Bündel durchlässt.

15. Gerät nach einem der Ansprüche 13 oder 14, dadurch gekennzeichnet, dass, wenn das Ziel ($C_2$) sich in der Stellung ($P_1$) befindet, das Ziel ($C_1$) auf dem Strahlenweg des Bündels ($f_1$) liegt und ein Röntgenstrahlenbündel ($f_2$) mit niedriger Energie in Richtung der YY-Achse abstrahlt.

16. Gerät nach einem der Ansprüche 13 oder 14, dadurch gekennzeichnet, dass bei eingezogenem Ziel ($C_1$) das Ziel ($C_2$) sich in der Stellung ($P_1$) befindet und das Bündel ($f_3$) stark beschleunigter geladener Teilchen abgestrahlt wird.

17. Gerät nach einem der Ansprüche 13 oder 14, dadurch gekennzeichnet, dass bei eingezogenem Ziel ($C_1$) das Ziel ($C_2$) sich in der Stellung ($P_2$) befindet, das Bündel ($f_3$) abgestrahlt wird und durch Bombardieren des Ziels ($C_2$) das Abstrahlen eines Röntgenstrahlenbündels ($f_4$) mit hoher Energie bewirkt.

## Claims

1. Radiographic device using the loaded particle accelerator of a radiotherapy apparatus designed to provide along a YY-axis an irradiation beam, said apparatus comprising a particle accelerator (A) including a source (S) of loaded particles which is adapted to supply a beam ($f_1$) of slightly accelerated loaded particles, an accelerating structure (A) allowing a beam ($f_3$) of highly accelerated loaded particles to be obtained, characterized in that it comprises a device (D) for magnetic deviation of loaded particles and at least one first target (C) adapted to be positioned at least temporarily in such a manner that it receives the slightly accelerated particle beam ($f_1$) and emits in the direction of the YY-axis a low energy X-ray beam ($f_2$).

2. Device according to claim 1, characterized in that said first target is a fixed target (C) located on the YY-axis, the surface plane of said target being inclined at an angle $\alpha$ with respect to the normal to said YY-axis, the target (C) being adapted, at a first stage, to be bombarded by the slightly accelerated loaded particle beam ($f_1$), so as to emit the low energy ray beam ($f_2$) and, at a second stage, to be bombarded by the highly accelerated loaded particle beam ($f_3$), so as to emit a high energy X-ray beam ($f_4$).

3. Device according to claim 2, characterized in that the magnetic deviation device (D) comprises at least two deviators ($d_1$, $d_2$) adapted to deviate the slightly accelerated loaded particle beam ($f_1$) issuing from the accelerator (A), first by an angle $\gamma_1$ and then by an angle $\gamma_2$, which angles are oriented in opposite directions.

4. Device according to claim 3, characterized in that the angle of incidence ($\beta$) of the deviated beam ($f_1$), as reaching the target (C), is greater than, or equal to $\alpha$.

5. Radiotherapy apparatus adapted to supply along a YY-axis an irradiation beam, comprising a particle accelerator (A) equipped with a loaded particle source (S) which is adapted to supply a slightly accelerated loaded particle beam ($f_1$), an accelerating structure (3) allowing a highly accelerated loaded particle beam ($f_3$) to be obtained, and a device (D) for magnetic deviation of loaded particles, characterized in that it further comprises at least one first target (C) adapted to be positioned at least temporarily in such a manner that it receives the slightly accelerated particle beam ($f_1$) and emits in the direction of the YY-axis a low energy X-ray beam ($f_2$).

6. Apparatus according to claim 5, characterized in that said first target is a fixed target (C) located on the YY-axis, the surface plane of said target being inclined by an angle $\alpha$ with respect to the normal to said YY-axis, the target (C) being adapted to be bombarded, at a first stage, by the slightly accelerated loaded particle beam ($f_1$), so as to emit the low energy X-ray beam ($f_2$) and, at a second stage, to be bombarded by the highly accelerated loaded particle beam ($f_3$), so as to emit the high energy X-ray beam ($f_4$).

7. Apparatus according to claim 6, characterized in that the magnetic deviation device (D) comprises at least two deviators ($d_1$, $d_2$) adapted to deviate first by an angle $\gamma_1$ and then by an angle $\gamma_2$ the slightly accelerated loaded particle beam ($f_1$) issuing from the accelerator (A), said angles being oriented in opposite directions.

8. Apparatus according to claim 7, characterized in that the angle of incidence ($\beta$) of the deviated beam ($f_1$), as reaching the target (C), is greater than, or equal to $\alpha$.

9. Apparatus according to claim 5, characterized in that the YY-axis forms an angle of 90° with the axis of the non-accelerated loaded particle beam ($f_1$) reaching the target (C).

10. Apparatus according to claim 9, characterized in that the deviation device (D) is of the type $\pi 2$, the axis of the loaded particle beam ($f_3$), when issuing from the deviation device (D), then coinciding with the YY-axis.

11. Apparatus according to claim 9, characterized in that the deviation device (D) is of the $3\pi/2$ type, the axis of the loaded particle beam ($f_3$), when issuing from the deviation device (D), then coinciding with the YY-axis.

12. Apparatus according to one of claims 9 or 10, characterized in that the first target is a retractable target ($C_1$) adapted to be positioned on the path of the beam ($f_1$) for emitting the low energy X-ray beam ($f_2$) oriented in the direction of the YY-axis.

13. Apparatus according to one of claims 10 to 12, characterized in that it further comprises a second target ($C_2$) located on the YY-axis and adapted to be brought into two positions ($P_1$) and ($P_2$).

14. Apparatus according to claim 13, characterized in that said target ($C_2$) has a circular shape and is provided with an excentric aperture (11) such that in the position ($P_2$) it performs its function as a target, while in the position ($P_1$) where the axis of the aperture (11) coincides with the YY-axis, it allows any beam emitted in the direction of said axis to pass freely.

15. Apparatus according to one of claims 13 or 14, characterized in that when said target ($C_2$) is in the position ($P_1$), the target ($C_1$) is located on the path of the beam ($f_1$) and emits a low energy X-ray beam ($f_2$) in the direction of the YY-axis.

16. Apparatus according to one of claims 13 or 14, characterized in that, when the target ($C_1$) is retracted, the target ($C_2$) is in the position ($P_1$) and the highly accelerated loaded particle beam is emitted.

17. Apparatus according to one of claims 13 or 14, characterized in that when the target ($C_1$) is retracted, the target ($C_2$) is in the position ($P_2$), the beam ($f_3$) is emitted and by bombarding the target ($C_2$) initiates the emission of a high energy X-ray beam ($f_4$).

$$\boxed{\mathsf{FIG}}. 1$$

Fig. 2

Fig. 4

Fig. 3